# EUROPÄISCHE PATENTSCHRIFT

(11) **EP 3 165 517 B1**
(45) Veröffentlichungstag und Bekanntmachung des Hinweises auf die Patenterteilung: **12.08.2020**
(21) Anmeldenummer: 16020058.0
(22) Anmeldetag: 29.02.2016
(51) Int. Cl.: C07C 29/149, C07C 31/125, B01J 8/00, B01J 8/04

(54) **ANLAGE UND VERFAHREN ZUR ERZEUGUNG VON FETTALKOHOL**
INSTALLATION AND METHOD FOR THE PRODUCTION OF FATTY ALCOHOL
INSTALLATION ET PROCEDE DE FABRICATION D'ALCOOLS GRAS

(30) Priorität: 06.11.2015 EP 15400052
(43) Veröffentlichungstag der Anmeldung: 10.05.2017
(73) Patentinhaber: L'Air Liquide Société Anonyme pour l'Etude et l'Exploitation des Procédés Georges Claude, 75007 Paris (FR)
(72) Erfinder: Pötschacher, Peter, 60594 Frankfurt am Main (DE); Hoffmann, Manfred, 61273 Wehrheim (DE); Pomrehn, Fredrik, 60388 Frankfurt am Main (DE); Brandner, Armin, 63329 Egelsbach (DE)
(74) Vertreter: Dropsch, Holger

(56) Entgegenhaltungen:
- WO-A1-02/100807
- US-A- 4 185 967
- US-A1- 2007 225 527
- US-A1- 2013 072 717

## Beschreibung

### Gebiet der Erfindung

Die Erfindung betrifft eine Anlage zur Durchführung eines kontinuierlichen Verfahrens zur Herstellung von Fettalkohol aus Fettsäureester durch katalytische Rieselbetthydrierung, umfassend mehrere, jeweils mindestens ein Katalysatorfestbett enthaltende Schachtreaktoren, die so miteinander über Rohrleitungen verbunden sind, dass sie vom Edukt-/Produktgemisch nacheinander durchströmt werden können.

Die Erfindung umfasst weiterhin ein Verfahren zum Betreiben der Anlage.

### Stand der Technik

Derartige Anlagen und Verfahren sind bekannt. In Ullmann's Encyclopedia of Industrial Chemistry, Sixth Edition, Vol. 13, Seite 115 ist dieses Verfahren als "Trickle-Bed Hydrogenation" grundsätzlich beschrieben. Dabei wird, unter erhöhtem Druck und bei erhöhter Temperatur, der umzuwandelnde Fettsäureester in flüssiger Form mit gasförmigem, stöchiometrisch im Überschuss zugegebenem Wasserstoff zu einem Eduktgemisch vermischt. Das Eduktgemisch wird über ein Katalysatorfestbett, das auch als Rieselbett bezeichnet werden kann, geleitet, wobei das Eduktgemisch in ein aus Fettalkohol, Wasserstoff und, je nach verwendetem Fettsäureester, auch weitere Alkohole bestehendes Produktgemisch umgewandelt wird. Das Produktgemisch wird auf eine Temperatur im Bereich von 40 bis 80 °C, bevorzugt 60 bis 80 °C abgekühlt. Dann werden der Wasserstoff und leicht flüchtige Komponenten aus dem Produktgemisch abgetrennt. Der flüssige Fettalkohol wird als Produkt zur weiteren Verwendung aus der Anlage ausgeleitet. Der Wasserstoff wird an den Anfang des Verfahrens zurückgeführt, wo er mit frischem Wasserstoff zusammengeführt wird und zur Bildung des Eduktgemischs wiederverwendet wird.

Zur Durchführung der katalytischen Umwandlung wird das Eduktgemisch mit einer Temperatur im Bereich von 150 bis 250 °C, bevorzugt 180 bis 250 °C dem Katalysatorfestbett (Rieselbett) aufgegeben. Die Hydrierung verläuft exotherm, wobei ein zu starker Anstieg der Temperatur des Edukt-/Produktgemischs vermieden werden muss, um die Bildung von unerwünschten Nebenprodukten so gering wie möglich zu halten.

Ein zu starker Anstieg der Temperatur kann auch zu einer Verringerung der Standzeit des Katalysators führen. Die Anmeldeschrift US 2007/0225527 A1 stellt daher ein Verfahren vor, bei dem das Katalysatorbett auf einen Haupt- und zwei Hilfsreaktoren aufgeteilt wird. Diese Reaktoren werden dabei so in Reihe geschaltet, dass die Temperaturspitze in einen der Hilfsreaktoren verlegt und der Katalysatorinhalt des Hauptreaktors so geschont wird und eine lange Standzeit erreicht.

In der deutschen Patentschrift DE 198 43 798 C2 wird daher eine Verfahrensvariante vorgeschlagen, bei der zur Begrenzung des Temperaturanstiegs Quench-Wasserstoff in das Katalysatorfestbett eingeleitet wird. Dabei wird der Quench-Wasserstoff erhalten, indem vom gekühlten, aus dem Produktgemisch abgetrennten Wasserstoff ein Teil als "Quench-Wasserstoff" abgezweigt wird. Der übrige Wasserstoff wird, mit Frisch-Wasserstoff vermischt, als Kreislaufwasserstoff an den Prozessanfang zurückgeführt und zur Bildung des Eduktgemischs verwendet. Außerdem wird dort vorgeschlagen, das Katalysatorfestbett aufzuteilen und in zwei hintereinander geschalteten Reaktoren anzuordnen und dabei in die Transferleitung zwischen den Reaktoren für das Edukt-/Produktgemisch ebenfalls Quench-Wasserstoff einzuspeisen.

Nachteilig an diesen Anlagen- und Verfahrenskonzepten ist es, dass zum Austausch des nach einer gewissen Betriebszeit in seiner Wirkung nachlassenden Katalysators die Anlage stillgelegt werden muss. Weiterhin ist es nachteilig, dass die Wirksamkeit des untersten Teils des Katalysatorbetts nicht vollständig genutzt werden kann.

### Beschreibung der Erfindung

Aufgabe der vorliegenden Erfindung ist es daher, eine Anlage und ein Verfahren zur Verfügung zu stellen, die die Nachteile des Standes der Technik überwinden. Die Aufgabe wird durch die Erfindung entsprechend der Merkmale der unabhängigen Ansprüche durch die erfindungsgemäße Anlage und das erfindungsgemäße Verfahren gelöst.

### Erfindungsgemäße Anlage:

Anlage zur kontinuierlichen Herstellung von Fettalkohol aus Fettsäureester, insbesondere Wachsester, durch katalytische Rieselbetthydrierung, umfassend Zuführleitungen für den Fettsäureester und Wasserstoff, eine Abführleitung für das flüssige, Fettalkohol enthaltende Produktgemisch, eine Rückführleitung für nicht umgesetzten Wasserstoff und mehrere, bevorzugt zwei, jeweils mindestens ein Katalysatorfestbett enthaltende Schachtreaktoren, die so miteinander über Rohrleitungen verbunden sind, dass sie vom Edukt-/Produktgemisch nacheinander durchströmt werden können, wobei die Anlage und die Reaktoren so gestaltet sind, dass unterhalb des oder der Katalysatorbetten und/oder in die Transferleitung zwischen den Reaktoren Quench-Wasserstoff eingespeist werden kann, dadurch gekennzeichnet, dass die Reaktoren rohrleitungsseitig so verschaltet sind, dass
a) die Reihenfolge, in der die Reaktoren vom Edukt-/Produktgemisch durchströmt werden, frei wählbar ist und
b) jeder Reaktor jeweils auch alleine, unter Umgehung des jeweils anderen Reaktors, durchströmt werden kann und
c) jeder der Reaktoren bezüglich des oder der darin enthaltenen Katalysatorfestbetten so ausgelegt ist, dass bei einer vorgegebenen Produktionsleistung der Anlage der dazu benötigte Umsetzungsgrad der Edukte mit dem Durchgang durch nur einen der Reaktoren mindestens für die Dauer eines Austauschs des oder der Katalysatorfestbetten im jeweils anderen Reaktor erreicht werden kann und
d), dadurch gekennzeichnet, dass in den Schachtreaktoren Temperaturmessgeräte zur Sichtbarmachung des Temperaturverlaufs über die Länge des Festbetts installiert sind.

Beim Quench-Wasserstoff handelt es sich um Wasserstoff, dessen Temperatur, beispielsweise 75°C, deutlich unterhalb der Temperatur liegt, bei der die Hydrierung durchgeführt wird. Durch seine Einspeisung gemäß Anspruch 2 kann der Temperaturanstieg in den Reaktoren begrenzt werden.

### Erfindunqsqemäßes Verfahren:

Verfahren zur kontinuierlichen Herstellung von Fettalkohol aus Fettsäureester, insbesondere Wachsester, durch katalytische Rieselbetthydrierung, umfassend die folgenden Verfahrensstufen:
a) Bereitstellen von Fettsäureester und von frischem und rückgeführtem Wasserstoff,
b) Herstellen eines Eduktgemischs aus Fettsäureester und Wasserstoff bei einem Druck im Bereich von 50 bis 250 bar, bevorzugt 75 bis 100 bar,
c) Erhitzen des Gemischs auf eine Temperatur im Bereich 150 bis 250 °C, bevorzugt 180 bis 250°C,
d) Durchleiten des Gemisch durch einen ersten und dann durch einen zweiten Schachtreaktor, die jeweils mindestens ein, als Rieselbett ausgeführtes Katalysatorfestbett enthalten, wobei es zur Umwandlung des Eduktgemischs in ein Produktgemisch kommt, das Fettalkohol, Wasserstoff und, je nach verwendetem Fettsäureester, auch weitere Alkohole enthält,
e) Kühlung des Produktgemischs,
f) Abtrennung des Wasserstoffs von dem flüssigen, Fettalkohol enthaltenden Produktgemisch,
g) Rückführung des Wasserstoffs zur Verwendung in Schritt b),
h) Ausleitung des flüssigen, Fettalkohol enthaltenden Produktgemischs aus dem Verfahren zur weiteren Behandlung,
dadurch gekennzeichnet, dass der Austausch des Katalysators ohne Betriebsunterbrechung erfolgt, indem in Schritt d) die Wanderung der Reaktionszone durch die Katalysatorfestbetten der Schachtreaktoren durch Ermittlung des jeweiligen, sich über die Bettlänge ausbildenden und sich zeitlich verändernden Temperaturprofils beobachtet wird und, wenn die Reaktionszone vom ersten in den zweiten Reaktor übergetreten ist und wenn die Wanderung der Reaktionszone im zweiten Reaktor soweit fortgeschritten ist, dass sich über ihr im Katalysatorfestbett eine inaktive Zone gebildet hat, deren Länge ausreicht, um als Schutzzone zum Abfangen von Katalysatorgiften zu dienen, der erste Reaktor außer Betrieb genommen wird, indem das Gemisch aus Schritt c) direkt in den zweiten Reaktor eingeleitet wird, dass der verbrauchte Katalysator im ersten Reaktor durch frischen Katalysator ersetzt und der Reaktor wieder in Betrieb genommen wird, indem er dem anderen Reaktor nachgeschaltet wird.

Im normalen Produktionsbetrieb wird die Anlage so betrieben, dass zwei hintereinandergeschaltete Reaktoren vom Edukt-/Produktgemisch durchströmt werden. Die Hydrierung erfolgt dabei am Anfang der Betriebszeit der mit frischem Katalysator befüllten Anlage im ersten der Reaktoren. Im Laufe der Betriebszeit des Katalysatorbetts wandert die Reaktionszone, d. h. die Zone in der die Hydrierungsreaktionen ablaufen, von oben nach unten durch das Bett. Der über der Reaktionszone liegende, deaktivierte, d. h. verbrauchte Teil des Katalysators wirkt dabei als Schutzzone für den noch aktiven Katalysator, indem er Katalysatorgifte abfängt. Durch über die Länge des Katalysatorbetts verteilte Temperaturmessgeräte kann beobachtet werden, wie die Reaktionszone im Katalysatorbett im Verlauf der Betriebszeit durch das Bett wandert. Wenn die Reaktionszone den unteren Bereich des Katalysatorbetts im ersten Reaktor erreicht hat, beginnt sie allmählich in den Anfangsbereich des im zweiten Reaktor angeordneten Bettes über zu treten. Wenn die Reaktionszone vollständig in den zweiten Reaktor übergetreten ist und wenn sich dort im oberen, d.h. im Eintrittsteil des Katalysatorbetts eine Schicht aus deaktiviertem Katalysator gebildet hat, wird der erste Reaktor stillgelegt. Die Bildung einer Schicht aus deaktiviertem Katalysator wird abgewartet, da sie als Schutzschicht gegen Katalysatorgifte dient. Wie lang diese Schutzschicht zu sein hat, um ausreichend wirksam zu sein, muss durch Betriebsversuche ermittelt werden.

Um den Katalysator des ersten Reaktors auszutauschen, wird nun das Edukt-/Produktgemisch nur noch über den zweiten Reaktor geleitet. Nach erfolgtem Katalysatoraustausch werden die Reaktoren, in umgekehrter Reihenfolge, wieder hintereinandergeschaltet und nacheinander durchströmt. Auf diese Weise wird der Katalysator über die gesamte Länge des Bettes vollständig ausgenutzt und der Austausch des verbrauchten Katalysators kann ohne Betriebsunterbrechung erfolgen. Außerdem ist durch diese Anordnung von zwei hintereinander geschalteten Reaktoren gewährleistet, dass auch wenn die Reaktionszone vom ersten in den zweiten Reaktor übertritt, das Edukt-/Produktgemisch stets erst durch eine Schutzschicht aus deaktiviertem Katalysator strömt, bevor es die Reaktionszone erreicht.

Außerhalb der erfindungsgemäßen Anlage und des erfindungsgemäßen Verfahrens wird überschüssiger, bei der Hydrierung nicht verbrauchter Wasserstoff auf mechanischem Weg aus dem Produkt abgetrennt und zur Wiederverwendung in die Anlage zurückgeführt.

### Bevorzugte Ausgestaltungen der Erfindung

Eine bevorzugte Ausgestaltung der Erfindung ist dadurch gekennzeichnet, dass sie eine Entspannungsvorrichtung umfasst, die mit der Zuführleitung für den Fettsäureester verbunden ist. Die Entspannungsvorrichtung kann mit einem System zum Absaugen von Gasen und/oder Dämpfen ausgestattet sein, durch die darin ein Druck zwischen 1 und 1000 mbar, bevorzugt zwischen 10 und 700 mbar erzeugt wird. Eine derartige Vorrichtung ist besonders vorteilhaft, wenn es sich bei dem Fettsäureester um einen Wachsester handelt. Durch den Einsatz der Entspannungsvorrichtung kann der Gehalt an freien Fettsäuren und sonstigen, gegenüber dem Wachsester, leichter flüchtigen Verbindungen im Fettsäure- bzw. Wachsester reduziert werden.

### Ausführungsbeispiel

Weitere Merkmale, Vorteile und Anwendungsmöglichkeiten der Erfindung ergeben sich auch aus der nachfolgenden Beschreibung von Ausführungs- und Zahlenbeispielen und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

### Es zeigt die einzige Figur

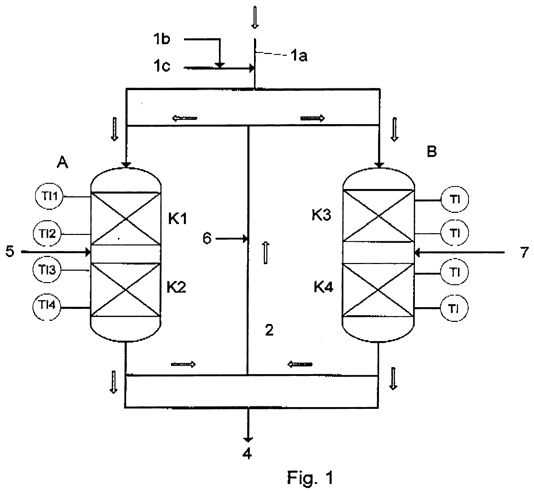

Fig. 1 ein Schema der rohrseitigen Verschaltung zweier katalysatorbefüllter Festbettreaktoren einer erfindungsgemäßen Anlage.

Die erfindungsgemäße Verschaltung soll an Hand der Fig. 1 im Folgenden erläutert werden.

Die Reaktoren A und B sind über das dargestellte Rohrleitungssystem miteinander verschaltet, d. h. verbunden. Rohrleitungsarmäturen, wie beispielsweise Ventile, sind nicht dargestellt. Durch Zuführleitung 1 wird das Eduktgemisch, bestehend aus einem flüssigen Fettsäureester und Wasserstoff, mit einem Druck im Bereich von 75 bis 100 bar und einer Temperatur von beispielsweise 180 °C in das System und in einen der Reaktoren eingeleitet. Die Reaktoren enthalten jeweils zwei Katalysatorfestbetten, K1 bis K4. Beim Durchrieseln der Katalysatorfestbetten wird das Eduktgemisch in ein Produktgemisch, bestehend aus Fettalkohol und Wasserstoff, umgewandelt. Die Hydrierung verläuft exotherm und führt zu einem Temperaturanstieg im Gemisch bzw. in der Reaktionszone des Katalysatorbetts. Durch in den Reaktoren installierte Temperaturmessgeräte, TI, wird der Verlauf der Temperatur über die Länge des Festbetts und damit die Wanderung der Reaktionszone durch das Festbett sichtbar. Im Produktionsmodus der Anlage wird das Gemisch über die Transferleitung 2 in den jeweils nachgeschalteten Reaktor weitergeleitet. Das Produktgemisch verlässt das System zur weiteren Behandlung in der Anlage über Leitung 4. Das Leitungssystem erlaubt auch, im Falle des Katalysatoraustauschs in einem der Reaktoren, den Durchfluss des Gemischs so zu führen, dass es nur einen der Reaktoren durchströmt und danach direkt zur Weiterbehandlung geleitet wird.

Über die Leitungen 5, 6 und 7 kann Quench-Wasserstoff in das Edukt-/Produktgemisch eingeleitet werden.

Das folgende Beispiel 1 zeigt, wie durch die Verwendung des Quench-Wasserstoffs die Menge des im Kreis geführten Wasserstoffs reduziert werden kann:

### Beispiel 1:

Bei der Hydrierung einer C₁₆/C₁₈-Wachsestertraktion sollen eine Reaktorzulauftemperatur von 180 °C und eine maximale Erwärmung von 30 °C eingehalten werden. Dazu wird der Fettsäureester (1a) mit Frischwasserstoff (1b) und Kreislaufwasserstoff (1c) vermischt und in den Hydrierreaktor mit vier Katalysatorbetten geleitet. Das Produkt (4) besteht überwiegend aus Fettalkohol und Wasserstoff. Die Ergebnisse wurden mittels Prozeßsimulation ermittelt.

Ohne Quenchgaskühlung wird dem Reaktor eine Kreislauf-Wasserstoffmenge von 6834 kg pro 25000 kg/h Wachsester zugeführt, während mit Quenchgaskühlung die Kreislauf-Wasserstoffmenge (H_{2,Krelslauf} + Summe H_{2,Quench}) auf 3621 kg/h pro 25000 kg/h Wachsester reduziert werden kann.

| Strom | Bezeichnung | Ohne Quenchgaskühlung | | Mit Quenchgaskühlung | |
|---|---|---|---|---|---|
| | | Massenfluss kg/h | Temperatur °C | Massenfluss kg/h | Temperatur °C |
| 1a | Fettsäuresester | 25000 | 232 | 25000 | 232 |
| 1b | H_{2,frisch} | 225 | 60 | 225 | 60 |
| 1c | H_{2,Kreislauf} | 6834 | 151 | 2676 | 105 |
| 4 | Produkt | 32059 | 210 | 28846 | 210 |
| 5 | H_{2,Quench 1} | - | - | 718 | 75 |
| 6 | H_{2,Quench 2} | - | - | 189 | 75 |
| 7 | H_{2,Quench 3} | - | - | 38 | 75 |
| Summe H₂ (Ströme 2+3+5+6+7) | | 6834 | | 3621 | |
| | | | | | |
| Tl1 | Bett 1 Kopf | - | 180 | - | 180 |
| Tl2 | Bett 1 Boden | - | 200 | - | 210 |
| Tl3 | Bett 2 Kopf | - | 200 | - | 199 |
| Tl4 | Bett 2 Boden | - | 208 | - | 210 |
| Tl5 | Bett 3 Kopf | - | 208 | - | 207 |
| Tl6 | Bett 3 Boden | - | 208 | - | 210 |
| Tl7 | Bett 4 Kopf | - | 210 | - | 209 |
| Tl8 | Bett 4 Boden | - | 210 | - | 210 |

Beispiel 2 zeigt die Abhängigkeit des Gehalts an Kohlenwasserstoffen im Produkt von der Reaktionstemperatur unter der die Hydrierung abläuft:

### Beispiel 2:

Bei der Hydrierung eines Wachsesters wurde der Gehalt an Kohlenwasserstoffen in Abhängigkeit der Reaktionstemperatur experimentell bestimmt (Tabelle 1). Bis zu einer Reaktortemperatur von 200 °C liegt der Kohlenwasserstoffgehalt bei unter 0.05 Gewichtsprozent. Oberhalb einer Temperatur von 200 °C steigt der Kohlenwasserstoffgehalt deutlich an.

**Tabelle 1**

| Reaktionstemperatur | Kohlenwasserstoffgehalt im Produkt |
|---|---|
| [°C] | [Gew-%] |
| 170 | 0 |
| 180 | 0,01 |
| 190 | 0,02 |
| 200 | 0,03 |
| 210 | 0,16 |
| 215 | 0,26 |
| 220 | 0,54 |

### Gewerbliche Anwendbarkeit

Die Erfindung stellt eine Anlage zur Verfügung, die auch während des Austausch des Katalysators unterbrechungsfrei weiter produzieren kann und die es ermöglicht, die Ausnutzung des Katalysators zu optimieren. Die Erfindung ist daher gewerblich anwendbar.

### Bezugszeichenliste

- 1 a: Zuführleitung für Fettsäureester
- 1 b: Zuführung für Frischwasserstoff
- 1c: Zuführung für rückgeführten Wasserstoff
- 2: Transferleitung
- 3: Unbesetzt
- 4: Abführleitung für Produktgemisch
- 5: Quench-Wasserstoffzufuhr
- 6: Quench-Wasserstoffzufuhr
- 7: Quench-Wasserstoffzufuhr
- A: Reaktor
- B: Reaktor
- K1: Katalysatorfestbett
- K2: Katalysatorfestbett
- K3: Katalysatorfestbett
- K4: Katalysatorfestbett
- Tl1 bis Tl8: Temperaturmessgerät

## Patentansprüche

1. Anlage zur kontinuierlichen Herstellung von Fettalkohol aus Fettsäureester, insbesondere Wachsester, durch katalytische Rieselbetthydrierung, umfassend Zuführleitungen für den Fettsäureester und frischen und für rückgeführten Wasserstoff, eine Abführleitung für das flüssige, Fettalkohol enthaltende Produktgemisch, eine Rückführleitung für nicht umgesetzten Wasserstoff und mehrere, bevorzugt zwei, jeweils mindestens ein Katalysatorfestbett enthaltende Schachtreaktoren, die so miteinander über Rohrleitungen verbunden sind, dass sie vom Edukt-/Produktgemisch nacheinander durchströmt werden können, wobei die Anlage und die Reaktoren so gestaltet sind, dass unterhalb des oder der Katalysatorbetten und/oder in die Transferleitung zwischen den Reaktoren Quench-Wasserstoff eingespeist werden kann, **dadurch gekennzeichnet, dass** die Reaktoren rohrleitungsseitig so verschaltet sind, dass
a) die Reihenfolge, in der die Reaktoren vom Edukt-/Produktgemisch durchströmt werden, frei wählbar ist und
b) jeder Reaktor jeweils auch alleine, unter Umgehung des jeweils anderen Reaktors, durchströmt werden kann und
c) jeder der Reaktoren bezüglich des oder der darin enthaltenen Katalysatorfestbetten so ausgelegt ist, dass bei einer vorgegebenen Produktionsleistung der Anlage der dazu benötigte Umsetzungsgrad der Edukte mit dem Durchgang durch nur einen der Reaktoren mindestens für die Dauer eines Austauschs des oder der Katalysatorfestbetten im jeweils anderen Reaktor erreicht werden kann und
d), **dadurch gekennzeichnet, dass** in den Schachtreaktoren Temperaturmessgeräte zur Sichtbarmachung des Temperaturverlaufs über die Länge des Festbetts installiert sind.

2. Anlage gemäß Anspruch 1, ferner umfassend eine Entspannungsvorrichtung, die mit der Zuführleitung für den Fettsäureester verbunden ist.

3. Verfahren zur kontinuierlichen Herstellung von Fettalkohol aus Fettsäureester, insbesondere Wachsester, durch katalytische Rieselbetthydrierung, umfassend die folgenden Verfahrensstufen:
a) Bereitstellen von Fettsäureester und von frischem und rückgeführtem Wasserstoff,
b) Herstellen eines Eduktgemischs aus Fettsäureester und Wasserstoff bei einem Druck im Bereich von 50 bis 250 bar, bevorzugt 75 bis 100 bar,
c) Erhitzen des Gemischs auf eine Temperatur im Bereich 150 bis 250 °C, bevorzugt 180 bis 250°C,
d) Durchleiten des Gemisch durch einen ersten und dann durch einen zweiten Schachtreaktor, die jeweils mindestens ein, als Rieselbett ausgeführtes Katalysatorfestbett enthalten, wobei es zur Umwandlung des Eduktgemischs in ein Produktgemisch kommt, das Fettalkohol, Wasserstoff und, je nach verwendetem Fettsäureester, auch weitere Alkohole enthält,
e) Kühlung des Produktgemischs,
f) Abtrennung des Wasserstoffs von dem flüssigen, Fettalkohol enthaltenden Produktgemisch,
g) Rückführung des Wasserstoffs zur Verwendung in Schritt b),
h) Ausleitung des flüssigen, Fettalkohol enthaltenden Produktgemischs aus dem Verfahren zur weiteren Behandlung,
**dadurch gekennzeichnet, dass** der Austausch des Katalysators ohne Betriebsunterbrechung erfolgt, indem in Schritt d) die Wanderung der Reaktionszone durch die Katalysatorfestbetten der Schachtreaktoren durch Ermittlung des jeweiligen, sich über die Bettlänge ausbildenden und sich zeitlich verändernden Temperaturprofils beobachtet wird und, wenn die Reaktionszone vom ersten in den zweiten Reaktor übergetreten ist und wenn die Wanderung der Reaktionszone im zweiten Reaktor soweit fortgeschritten ist, dass sich über ihr im Katalysatorfestbett eine inaktive Zone gebildet hat, deren Länge ausreicht, um als Schutzzone zum Abfangen von Katalysatorgiften zu dienen, der erste Reaktor außer Betrieb genommen wird, indem das Gemisch aus Schritt c) direkt in den zweiten Reaktor eingeleitet wird, dass der verbrauchte Katalysator im ersten Reaktor durch frischen Katalysator ersetzt und der Reaktor wieder in Betrieb genommen wird, indem er dem anderen Reaktor nachgeschaltet wird.

4. Verfahren gemäß Anspruch 3, **dadurch gekennzeichnet, dass** der Fettsäureester ein Wachsester ist und dieser vor dem Verfahrensschritt 4 b) durch eine Entspannungsvorrichtung geleitet wird, um seinen Gehalt an freien Fettsäuren und sonstigen gegenüber dem Wachsester leichter flüchtigen Verbindungen zu reduzieren.

5. Verfahren gemäß Anspruch 3, **dadurch gekennzeichnet, dass** die Entspannungsvorrichtung bei Drücken zwischen 1 und 1000 mbar, bevorzugt zwischen 10 und 700 mbar betrieben wird.

## Claims

1. Plant for the continuous production of fatty alcohol from fatty acid ester, in particular wax ester, by catalytic trickle-bed hydrogenation, comprising supply conduits for the fatty acid ester and fresh and for recirculated hydrogen, a discharge conduit for the liquid product mixture containing fatty alcohol, a return conduit for non-converted hydrogen, and several, preferably two, shaft reactors each containing at least one catalyst fixed bed, which are connected with each other via pipe conduits such that they can be traversed by the educt/product mixture one after the other, wherein the plant and the reactors are configured such that quench hydrogen can be fed below the catalyst bed(s) and/or into the transfer conduit between the reactors, **characterised in that** the reactors are connected with each other by pipelines such that
a) the order in which the reactors are traversed by the educt/product mixture is freely selectable and
b) each reactor can also each be traversed alone, bypassing the respective other reactor and
c) each of the reactors is configured with respect to the catalyst fixed bed(s) contained therein such that at a specified production output of the plant, the required degree of conversion of the educts can be achieved with the passage through only one of the reactors at least for the duration of an exchange of the catalyst fixed bed(s) in the respective other reactor and
d) **characterised in that** temperature measurement devices for visualisation of the temperature profile over the length of the fixed bed are installed in the shaft reactors.

2. Plant according to claim 1, further comprising a depressurising device which is connected to the supply conduit for the fatty acid ester.

3. Process for the continuous production of fatty alcohol from fatty acid ester, in particular wax ester, by catalytic trickle-bed hydrogenation, said process comprising the following process steps:
a) providing fatty acid ester and fresh and recirculated hydrogen,
b) producing an educt mixture of fatty acid ester and hydrogen at a pressure in the range of 50 to 250 bar, preferably 75 to 100 bar,
c) heating the mixture to a temperature in the range of 150 to 250°c, preferably 180 to 250°C,
d) passing the mixture through a first and then through a second shaft reactor, which each contain at least one catalyst fixed bed in the form of a trickle bed, wherein the educt mixture is converted into a product mixture which contains fatty alcohol, hydrogen and, depending on the fatty acid ester used, also further alcohols,
e) cooling the product mixture,
f) separating the hydrogen from the liquid product mixture containing fatty alcohol,
g) recirculating the hydrogen for use in step b),
h) discharging the liquid product mixture containing fatty alcohol from the process for further treatment,
**characterised in that** the exchange of the catalyst is effected without interrupting operation, by , in step d), migration of the reaction zone through the catalyst fixed beds of the shaft reactors being monitored by determining the respective temperature profile forming along the bed length and varying in time and, when the reaction zone has crossed over from the first into the second reactor and when migration of the reaction zone in the second reactor has progressed to such an extent that an inactive zone has formed above said reaction zone in the catalyst fixed bed, the length thereof being sufficient to serve as a protection zone for trapping catalyst poisons, the first reactor is taken out of operation by introducing the mixture from step c) directly into the second reactor, that the consumed catalyst in the first reactor is replaced by fresh catalyst, and the reactor is again brought into operation by connecting it downstream of the other reactor.

4. Process according to claim 3, **characterised in that** the fatty acid ester is a wax ester and, before process step 4 b), is passed through a depressurising device in order to reduce its content of free fatty acids and other compounds of higher volatility than the wax ester.

5. Process according to claim 3, **characterised in that** the depressurising device is operated at pressures between 1 and 1000 mbar, preferably between 10 and 700 mbar.

## Revendications

1. Installation destinée à la production continue d'alcool gras à partir d'ester d'acide gras, en particulier d'ester cireux, par hydrogénation catalytique à lit ruisselant, comprenant des conduites d'alimentation pour l'ester d'acide gras et frais et pour de l'hydrogène recyclé, une conduite d'évacuation pour le mélange de produits liquide contenant un alcool gras, une conduite de recyclage pour de l'hydrogène non transformé et plusieurs, de préférence deux, réacteurs à puits contenant respectivement au moins un lit fixe de catalyseur, qui sont reliés entre eux par l'intermédiaire de canalisations de telle sorte qu'ils peuvent être traversés successivement par le mélange de produits de départ/mélange de produits, l'installation et les réacteurs étant configurés de telle sorte que de l'hydrogène de trempe peut être introduit en dessous du ou des lits catalytiques et/ou dans la conduite de transfert entre les réacteurs, **caractérisée en ce que** les réacteurs sont raccordés du côté des canalisations de telle sorte que
a) l'ordre, dans lequel les réacteurs sont traversés par le mélange de produits de départ/mélange de produits, peut être choisi librement et
b) chaque réacteur respectivement également seul, en contournant respectivement l'autre réacteur, peut être traversé et
c) chacun des réacteurs concernant le ou les lits fixes de catalyseurs contenus dans ceux-ci est conçu de telle sorte que lors d'un rendement prédéterminé de l'installation le degré de transformation des produits de départ requis à cet effet peut être atteint avec le passage à travers seulement un des réacteurs au moins pendant la durée d'un échange du ou des lits fixes de catalyseurs dans respectivement l'autre réacteur et
d) **caractérisée en ce que** des appareils de mesure de la température sont installés dans les réacteurs à puits pour visualiser l'évolution de la température sur la longueur du lit fixe.

2. Installation selon la revendication 1, comprenant en outre un dispositif détendeur, qui est relié avec la conduite d'alimentation pour l'ester d'acide gras.

3. Procédé destiné à la production continue d'alcool gras à partir d'ester d'acide gras, en particulier d'ester cireux, par hydrogénation catalytique à lit ruisselant, comprenant les étapes de procédé suivantes :
a) la fourniture d'ester d'acide gras et d'hydrogène frais et recyclé,
b) la production d'un mélange de produits de départ à partir d'ester d'acide gras et d'hydrogène sous une pression située dans la plage de 50 à 250 bars, de préférence de 75 à 100 bars,
c) le chauffage du mélange à une température située dans la plage de 150 à 250 °C, de préférence de 180 à 250 °C,
d) le passage du mélange à travers un premier et puis à travers un second réacteur à puits, qui contiennent respectivement au moins un lit fixe de catalyseur réalisé en tant que lit ruisselant, dans lequel cela aboutit à la transformation du mélange de produits de départ en un mélange de produits, qui contient de l'alcool gras, de l'hydrogène et, selon l'ester d'acide gras utilisé, également d'autres alcools,
e) le refroidissement du mélange de produits,
f) la séparation de l'hydrogène du mélange de produits liquide contenant l'alcool gras,
g) le recyclage de l'hydrogène pour l'utilisation à l'étape b),
h) l'évacuation du mélange de produits liquide contenant l'alcool gras à partir du procédé pour un traitement ultérieur,
**caractérisé en ce que** l'échange du catalyseur sans interruption de fonctionnement s'effectue, en surveillant à l'étape d) la migration de la zone de réaction par les lits fixes de catalyseurs des réacteurs à puits par détermination du profil de température respectif formé sur la longueur des lits et variable dans le temps et, lorsque la zone de réaction est passée du premier au second réacteur et lorsque la migration de la zone de réaction est avancée dans le second réacteur à tel point qu'une zone inactive s'est formée sur lui dans le lit fixe de catalyseur, présentant une longueur suffisante pour servir de zone de protection pour absorber des poisons de catalyseurs, le premier réacteur est mis hors service, en introduisant le mélange de l'étape c) directement dans le second réacteur, de manière à ce que le catalyseur utilisé dans le premier réacteur est remplacé par du catalyseur frais et le réacteur est remis en service, en étant branché à l'autre réacteur.

4. Procédé selon la revendication 3, **caractérisé en ce que** l'ester d'acide gras est un ester cireux et celui-ci est dirigé avant l'étape de procédé 4 b) par un dispositif détendeur pour diminuer sa teneur en acides gras libres et autres composés volatils plus légers par rapport à l'ester cireux.

5. Procédé selon la revendication 3, **caractérisé en ce que** le dispositif détendeur est actionné sous des pressions comprises entre 1 et 1000 mbars, de préférence entre 10 et 700 mbars.
